# EUROPEAN PATENT APPLICATION

(11) **EP 4 205 601 A1**
(43) Date of publication of application: **05.07.2023**
(21) Application number: 21867190.7
(22) Date of filing: 14.09.2021
(51) Int. Cl.: A46B 15/00

(54) **ELECTRIC TOOTHBRUSH**

(30) Priority: 14.09.2020 KR 20200117974
(71) Applicant: Proxihealthcare Inc., Ulsan 44988 (KR)
(72) Inventor: KIM, Young Wook, Seoul 03741 (KR)
(74) Representative: AWA Sweden AB
(86) International application number: PCT/KR2021/012451
(87) International publication number: WO 2022/055325

(57) **Abstract**

The present invention relates to an electric toothbrush that includes a head part and a handle part having a shape connectable to the head part and supplying a driving voltage to the head part according to a user's control. The head part may include a toothbrush head formed with a plurality of bristle holes where toothbrush bristles are disposed and a first through hole and a second through hole spaced apart from each other, a head body extending from the toothbrush head and having an inner passage connected to the first through hole and the second through hole, a head cover which closes the inner passage of the head body, and a first electrode rod and a second electrode rod inserted through the inner passage of the head body, whose one ends being positioned in the first through hole and the second through hole, respectively, and the other ends being exposed to the outside through the head cover, respectively.

## Description

### TECHNICAL FIELD

The present invention relates to an electric toothbrush, and more particularly, to an electric toothbrush capable of effectively removing dental plaque.

### BACKGROUND ART

Dental plaque is a sticky and transparent film that adheres to the surface of teeth. The dental plaque is formed as numerous germs (bacteria) living in the mouth adhered to certain components in saliva. The dental plaque may be formed not only on and around the teeth, but also around prostheses, braces, and dentures.

When the dental plaque in the form of a very thin and transparent film is created, the bacteria in the plaque proliferate also increase exponentially using the sugar supplied when food is consumed. The acidic substances produced by the bacteria in the plaque dissolve the lime in the teeth, causing tooth decay, and the toxins cause inflammation in the gums.

The dental plaque itself is difficult to see with the naked eye, and it mainly occurs in deep valleys of teeth, narrow gaps between teeth, and narrow gaps between teeth and gums. Because the plaque causes problems to teeth and surrounding tissues in such a small space, it is important to remove the plaque without missing every corner, but there is a problem in that it is difficult to effectively remove such plaque using only a conventional toothbrush.

### DISCLOSURE

### TECHNICAL PROBLEM

In order to solve the above problems, the present invention is to provide an electric toothbrush capable of effectively removing the dental plaque from humans or companion animals to solve the above problems.

In addition, the present invention is to provide an electric toothbrush capable of preventing tooth decay and periodontal disease through a removal of the dental plaque.

In addition, the present invention is to provide an electric toothbrush capable of preventing discomfort such as foreign body sensation that may occur to a user because electrodes providing an electric field do not protrude outside the toothbrush head.

In addition, the present invention is to provide an electric toothbrush capable of easily removing foreign substances present in a through hole or an electrode rod by disposing the electrode rod in the through hole formed in the toothbrush head.

### TECHNICAL SOLUTION

An electric toothbrush according to an embodiment of the present invention may include a head part and a handle part having a shape connectable to the head part and supplying a driving voltage to the head part according to a user's control, wherein the head part may include a toothbrush head formed with a plurality of bristle holes where toothbrush bristles are disposed and a first through hole and a second through hole spaced apart from each other, a head body extending from the toothbrush head and having an inner passage connected to the first through hole and the second through hole, a head cover which closes the inner passage of the head body, and a first electrode rod and a second electrode rod inserted through the inner passage of the head body, whose one ends being positioned in the first through hole and the second through hole, respectively, and the other ends being exposed to the outside through the head cover, respectively.

In addition, at least one row of bristle holes may be disposed between the first through hole and the second through hole.

In addition, the width of the first electrode rod and the second electrode rod may be set to 0.1 mm to 7 mm.

In addition, one end of the first electrode rod and one end of the second electrode rod may be exposed to the outside from the front and rear surfaces of the toothbrush head through the first through hole and the second through hole, respectively.

In addition, one end of the first electrode rod and one end of the second electrode rod may be characterized in that they do not protrude toward the outside relative to the front and rear surfaces of the toothbrush head.

In addition, the handle part may include a battery, a switch for controlling power supply by the battery, a circuit unit for generating a driving voltage using the voltage of the battery, and a first connection pin and a second connection pin contacting the other end of the first electrode rod and the other end of the second electrode rod, respectively when the head part and the handle part are coupled, in order to transfer the driving voltage generated in the circuit unit to the head part.

In addition, the driving voltage generated by the circuit unit may be set to a frequency of 1 KHz to 1,000 MHz.

In addition, the circuit unit may include a DC-DC converter receiving the voltage of the battery, a signal generator generating a driving voltage using the output voltage of the DC-DC converter, a filter performing a filtering operation on the driving voltage generated by the signal generator, and a calibration unit which performs and outputs voltage calibration for the driving voltage supplied from the filter.

In addition, the handle part may further include an inner case including a battery accommodating part in which the battery is located and a coupling part in which the first connection pin and the second connection pin are installed, and a circuit board where the circuit unit is mounted and fixed to the inner case.

In addition, the electric toothbrush may further include an upper cover made of an insulating material installed on the coupling part of the inner case and having a first opening and a second opening for exposing the first connection pin and the second connection pin to the outside, respectively, an outer case accommodating the inner case and having a switch pressing area corresponding to the switch, and a battery cap coupled to one end of the outer case to close the battery accommodating part.

In addition, an accommodating groove and a first wall part located inside the accommodating groove and connected to the inner passage may be formed at the distal end of the head body.

In addition, the head cover may be fused to the first wall part.

In addition, at least one first protrusion may be formed in the head cover, and at least one insertion hole to which the first protrusion is coupled may be formed in the first wall part.

In addition, a second wall part extended outward and having at least one second protrusion formed on the outer circumferential surface is formed at one end of the outer case, at least one insertion groove where the second protrusion is inserted and coupled may be formed on an inner surface of the distal end of the head body.

In addition, the driving voltage may be generated by overlapping an AC voltage having amplitude of A volts (V) and a DC voltage of B volts (V).

In addition, a ratio of the A and the B may be set to 1:0.5 to 10.

### ADVANTAGEOUS EFFECTS

According to the present invention, it is possible to provide an electric toothbrush capable of effectively removing the dental plaque from humans or companion animals.

In addition, according to the present invention, it is possible to provide an electric toothbrush capable of preventing an occurrence of tooth decay and periodontal disease through the removal of the dental plaque.

In addition, according to the present invention, it is possible to provide an electric toothbrush capable of preventing discomfort such as foreign body sensation that may occur to a user because the electrode providing an electric field does not protrude outside the toothbrush head.

In addition, according to the present invention, it is possible to provide an electric toothbrush capable of easily removing foreign substances present in the through hole or the electrode rod by disposing the electrode rod in the through hole formed in the toothbrush head.

### BRIEF DESCRIPTION OF THE DRAWINGS

FIG. 1 is a view showing an electric toothbrush according to an embodiment of the present invention.
FIG. 2 is a view showing a separated state of the electric toothbrush shown in FIG. 1.
FIG. 3 is a view showing an exploded state of a head part according to an embodiment of the present invention.
FIG. 4 is a view showing a coupled state of a head part according to an embodiment of the present invention.
FIG. 5 is a view showing the rear side of a toothbrush head according to an embodiment of the present invention.
FIG. 6 is a view showing a partial cross-section of a head part according to an embodiment of the present invention.
FIG. 7 is a view showing a handle part according to an embodiment of the present invention.
FIG. 8 is a view showing an exploded state of a handle part according to an embodiment of the present invention.
FIG. 9 is a view showing an exploded state of an inner case according to an embodiment of the present invention.
FIG. 10 is a view showing a coupling method of an electric toothbrush according to another embodiment of the present invention.
FIG. 11 is a diagram showing a circuit unit according to an embodiment of the present invention.
FIGS. 12a to 12c are diagrams for explaining driving voltages according to an embodiment of the present invention.

### BEST MODE

Hereinafter, embodiments of the present invention will be described in detail with reference to the accompanying drawings. However, the present invention is not limited to the embodiments disclosed below and may be implemented in various different forms. Also, it should be understood that all modifications, equivalents, or replacements thereof are included within the subject matter and scope of the present invention.

In describing elements of the present invention, terms such as first, second, A, B, (a), and (b) may be used. These terms are only used to distinguish one element from other elements, and the nature, sequence, or order of that element is not limited by the term. Further, it should be understood in this specification that if an element is described as being "connected", "combined", or "coupled" to/with any other element, the element may be directly connected, combined, or coupled to/with the other element, but another element may also be connected, combined, or coupled between both elements. In the case of being "connected", "combined", or "coupled", it may be understood as being physically or electrically connected, combined, or coupled, but is also electrically "connected", "combined", or "coupled" " as needed..

Hereinafter, an electric toothbrush according to embodiments of the present invention will be described with reference to drawings related to the embodiments of the present invention.

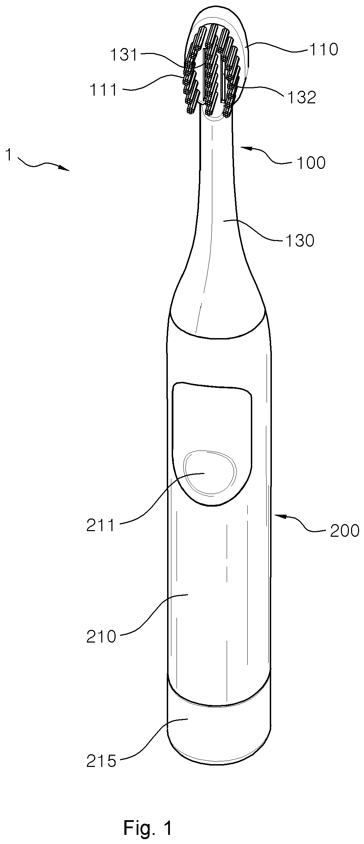

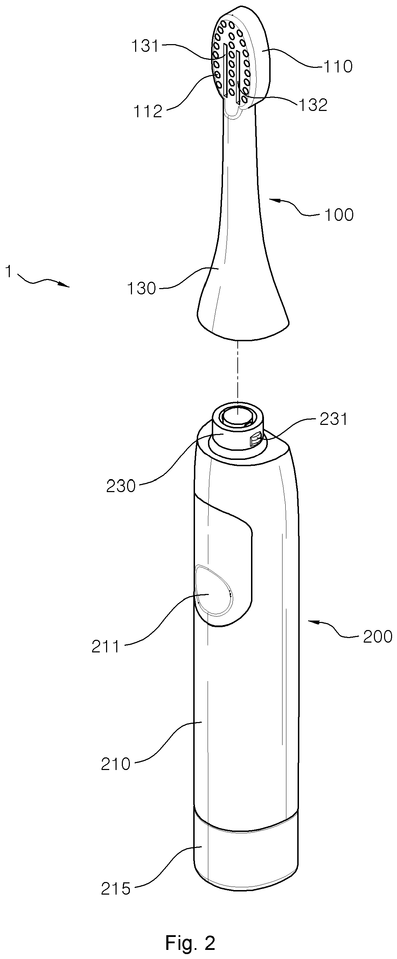

FIG. 1 is a view showing an electric toothbrush according to an embodiment of the present invention, and FIG. 2 is a view showing a separated state of the electric toothbrush shown in FIG. 1.

Referring to FIGS. 1 and 2, an electric toothbrush 1 according to an embodiment of the present invention may comprise a head part 100 and a handle part 200.

The head part 100 may include a toothbrush head 110 and a head body 130, and may be designed in a form capable of being combined with and separated from the handle part 200.

Accordingly, when replacement of the head part 100 is required due to deterioration or the like, a user can easily replace an existing head part 100 with a new head part 100.

The toothbrush head 110 may have bristles 111, a first electrode rod 131, and a second electrode rod 132 disposed thereon.

The bristles 111 may be inserted into and fixed to a plurality of bristle holes 112 formed on the surface of the toothbrush head 110. The arrangement structure, number, size, etc. of these bristles 111 are not particularly limited and may be changed in various forms.

The first electrode rod 131 and the second electrode rod 132 may be disposed on the toothbrush head 110 while being spaced apart from each other.

The first electrode rod 131 and the second electrode rod 132 may form an electric field based on electric energy supplied from the handle part 200. Since this electric field can weaken the dental plaque structure, the user can effectively remove the dental plaque in the oral cavity using the electric toothbrush 1.

The head body 130 may be extended from the toothbrush head 110 to form the body of the head part 100. The head body 130 may be designed to have a length suitable for use, and a distal end of the head body 130 may be coupled to the handle part 200.

The handle part 200 is a main body of the electric toothbrush 1 and may be designed in a form that the user can hold it when in use.

In addition, the handle part 200 may be combined with the head part 100 and may have a separate component for fixed coupling with the head part 100.

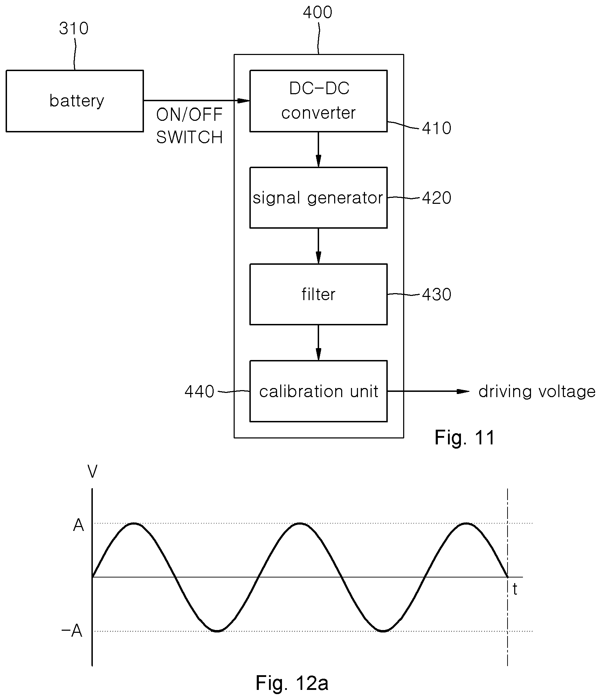

A battery 310 (see FIG. 11) for supplying power may be accommodated inside the handle part 200, and a separate battery cap 215 for replacement of the battery 310 may be provided on the handle part 200.

In addition, the handle part 200 may include an outer case 210 for accommodating and protecting inner components, and a switch pressing area 211 may be formed in the outer case 210.

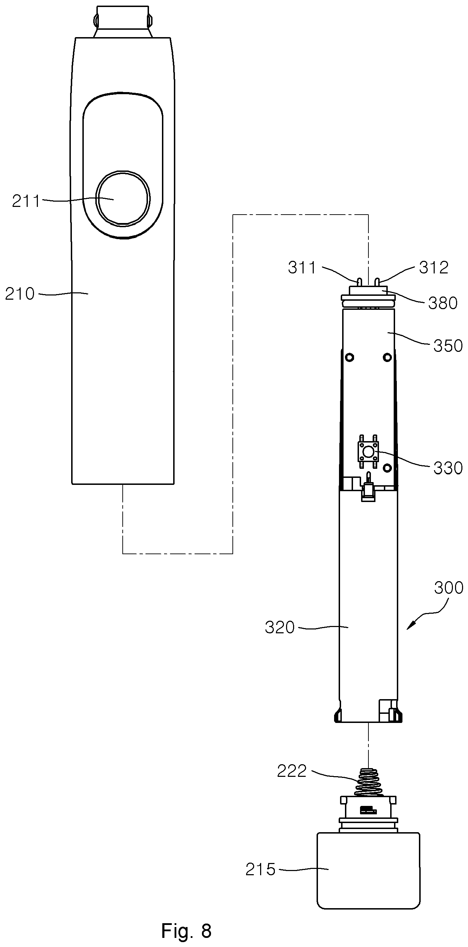

The switch pressing area 211 is formed at a position corresponding to the switch 330 (see FIG. 8) disposed therein, and the user can control on/off of the internal switch 330 by pressing the switch pressing area 211.

The user can turn on the power of the electric toothbrush 1 by pressing the switch pressing area 211 when brushing teeth, and thus the driving voltage generated from the handle part 200 is supplied to the first electrode rod 131 and/or the second electrode rod 132 of the head part 100 to generate an electric field for dental plaque removal.

In addition, a second wall part 230 extending outward may be formed at one end of the outer case 210, and at least one second protrusion 231 may be formed on an outer circumferential surface of the second wall part 230.

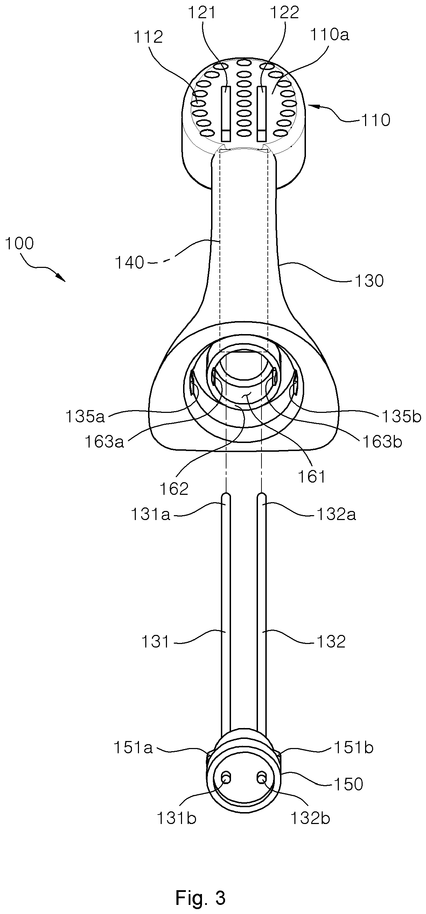

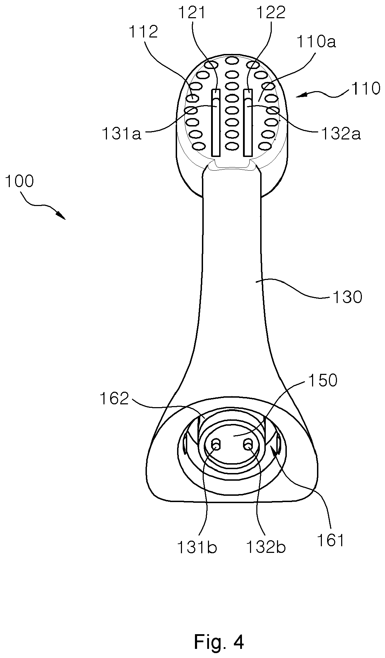

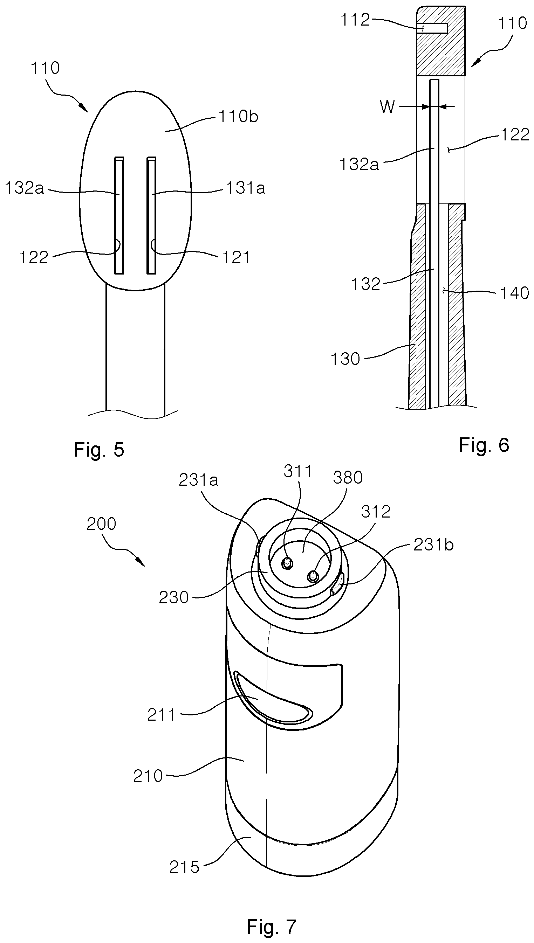

FIG. 3 is a view showing an exploded state of the head part according to an embodiment of the present invention, and FIG. 4 is a view showing a coupled state of the head part according to an embodiment of the present invention. FIG. 5 is a view showing the back side of the toothbrush head according to an embodiment of the present invention, and FIG. 6 is a view showing a partial cross section of the head part according to an embodiment of the present invention.

Referring to FIG. 3, a plurality of bristle holes 112 where the bristles 111 are disposed and a first through hole 121 and a second through hole 122 spaced apart from each other may be formed in the toothbrush head 110 according to the embodiment of the present invention.

The bristle holes 112 may be formed on the front surface 110a of the toothbrush head 110, and at least one row of bristle holes 112 may be placed between the first through hole 121 and the second through hole 122.

The first through hole 121 and the second through hole 122 may be formed to be elongated along the longitudinal direction (for example, vertical direction) of the head part 100.

The head body 130 may extend long from the toothbrush head 110 in a downward direction, and an inner passage 140 connected to the first through hole 121 and the second through hole 122 may be formed inside the head body 130. For example, one end of the inner passage 140 is connected to the first through hole 121 and the second through hole 122, and the other end of the inner passage 140 is opened at the distal end of the head body 130. The other end of the inner passage 140 may be coupled to a head cover 150 later.

The first electrode rod 131 and the second electrode rod 132 may be formed to be elongated along the length direction (for example, vertical direction) of the head part 100 and have a long straight rod or bar shape.

The first electrode rod 131 and the second electrode rod 132 may be installed in a state of being partially fixed to the head cover 150 in a state of being spaced apart from each other, and the other end 131b of the first electrode rod 131 and the other end 132b of the second electrode rod 132 may pass through the head cover 150 and be exposed to the outside, respectively.

The first electrode rod 131 and the second electrode rod 132 may be inserted into the head body 130 through the inner passage 140, such that one end 131a of the first electrode rod 131 and one end 132a of the second electrode rod 132 may be located in the first through hole 121 and the second through hole 122, respectively.

Since the first through hole 121 and the second through hole 122 have a shape penetrating the toothbrush head 110, one end 131a of the first electrode rod 131 and one end 132a of the second electrode rod 132 located in the first through hole 121 and the second through hole 122 may both be exposed to the outside from the front surface 110a and the rear surface 110b of the toothbrush head 110.

When one side of the through holes 121 and 122 accommodating the electrode rods 131 and 132 is closed, foreign substances caused by the use of the electric toothbrush 1 enter the through holes 121 and 122 and accumulate therein, thereby increasing the risk of bacterial growth. However, when the through holes 121 and 122 are opened to both sides of the toothbrush head 110 as in the embodiment of the present invention, the space in which foreign substances are accumulated is not only removed, but also the user can easily clean the inside of the through holes 121 and 122 and the electrode rods 131 and 132 using running water.

In addition, since one end 131a of the first electrode rod 131 and one end 132b of the second electrode rod 132 are located inside the first through hole 121 and the second through hole 122, they may not protrude outward based on the front surface 110a and the rear surface 110b of the toothbrush head 110. Through this configuration, it is possible to prevent discomfort such as foreign body sensation caused by the electrode rods 131 and 132 protruding outward from the toothbrush head 110.

In this case, the head cover 150 may close the inner passage 140 of the head body 130.

For the coupling of the head body 130 and the head cover 150, a separate first wall part 162 may be formed at the distal end of the head body 130. For example, a circular accommodating groove 161 may be formed at the distal end of the head body 130, and the first wall part 162 connected to the inner passage 140 may be placed inside the accommodating groove 161.

That is, the first wall part 162 may be formed in a wall shape surrounding the inlet side of the inner passage 140, and at least one insertion hole 163a, 163b may be formed in the first wall part 162.

Correspondingly, at least one first protrusion 151a, 151b may be formed in the head cover 150, and the first protrusions 151a and 151b of the head cover 150 are fastened to the insertion holes 163a and 163b of the first wall part 162, respectively, thus the head cover 150 may be coupled to the head body 130 as shown in FIG. 4,

In general, the head cover 150 may be fused to the first wall part 162 of the head body 130. That is, when separate insertion holes 163a and 163b are not formed in the first wall part 162, the head cover 150 may be coupled to the head body 130 through a fusion process using ultrasonic wave, vibration, heat, or the like.

In addition, at least one insertion groove 135a, 135b corresponding to the second projections 231a, 231b of the second wall part 230 described above may be formed on the inner surface of the distal end of the head body 130 adjacent to the accommodating groove 161.

Referring to FIG. 6, the first electrode rod 131 and the second electrode rod 132 may be designed to have the same width W as a whole. For example, the width W of the first electrode rod 131 and the second electrode rod 132 may be set to 0.1 mm to 7 mm.

When the width W of the electrode rods 131 and 132 is set to less than 0.1 mm, the corresponding electrode rods 131 and 132 are difficult to process and have low strength, and thus there is a possibility that contact failure may occur with the connection pins 311 and 312 of the handle part 200 (see FIG. 7). In addition, when the width W of the electrode rods 131 and 132 exceeds 7 mm, structural difficulties exist in placing them inside the toothbrush head 110 in consideration of the thickness of the toothbrush head 110.

On the other hand, when the electric toothbrush 1 is driven, the first electrode rod 131 and the second electrode rod 132 may be set as positive and negative electrodes, respectively. In addition, the first electrode rod 131 and the second electrode rod 132 may be formed of any materials such as brass, aluminum, conducting polymer, conducting silicon, stainless steel, or the like, but are not limited thereto, and any materials having conductivity may be used as the corresponding electrode material.

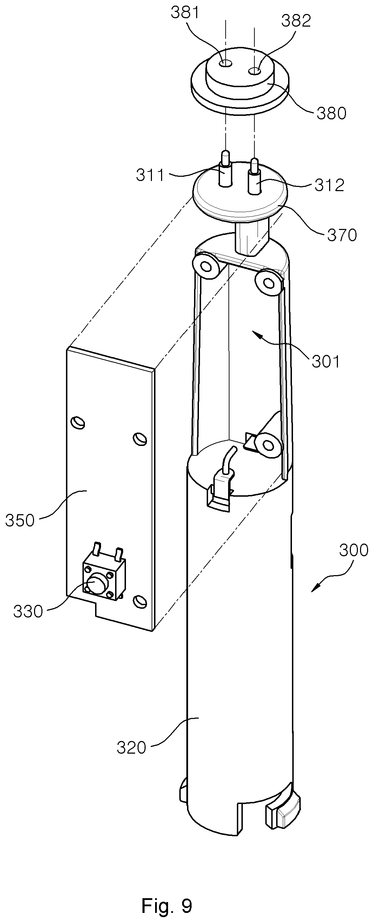

FIG. 7 is a view showing a handle part according to an embodiment of the present invention, FIG. 8 is a view showing an exploded state of the handle part according to an embodiment of the present invention, and FIG. 9 is a view showing an exploded state of the inner case according to an embodiment of the present invention.

Referring to FIGS. 7 to 9, the handle part 200 according to an embodiment of the present invention may include an inner case 300, a battery 310 (see FIG. 11), a first connection pin 311, and a second connection pin 312, a switch 330, a circuit board 350, and an upper cover 380.

The inner case 300 is accommodated inside the outer case 210 and may provide an area and space for installing components such as the battery 310, the switch 330, and the circuit board 350.

A battery accommodating part 320 in which a battery 310 is located is located at the bottom of the inner case 300, and a circuit board seating part 301 in which a circuit board 350 is seated on the upper side of the battery accommodating part 320 may be provided. In addition, a plate shaped coupling part 370 in which the connection pins 311 and 312 and the upper cover 380 are installed may be formed on the upper part of the inner case 300.

A battery terminal electrically connected to the circuit board 350 may be installed in the battery accommodating part 320, and the battery 310 may be inserted into the battery accommodating part 320 and electrically connected to the battery terminal.

For example, the battery 310 may be set as a primary battery or a secondary battery.

When the battery 310 is a primary battery, the user may periodically replace the battery 310, and when the battery 310 is a secondary battery, charging may be performed through various charging methods.

For example, the battery 310 may be charged through a wireless charging method or a wired charging method while being located in the battery accommodating part 320, and may be separated from the battery accommodating part 320 and charged through a separate charging device.

The switch 330 is for controlling power supply by the battery 310 and may be installed on the circuit board 350.

The circuit board 350 may be fixedly installed to the inner case 300, and for example, may be fixedly installed to the circuit board seating part 301 through a separate fastening member.

In addition, a circuit unit 400 (see FIG. 11) generating a driving voltage using the voltage of the battery 310 may be mounted on the circuit board 350.

The circuit unit 400 may generate a driving voltage using the voltage of the battery 310 supplied when the switch 330 is turned on.

The first connection pin 311 and the second connection pin 312 are each disposed in a form penetrating the coupling part 370, and when the head part 100 and the handle part 200 are coupled, may be in contact with the first electrode rod 131 and the second electrode rod 132 of the head part 100, respectively, so to serve to transfer the driving voltage generated in the circuit unit 400 to the head part 100.

To this end, lower ends of the connection pins 311 and 312 protruding downward of the coupling part 370 may be connected to the circuit board 350 on which the circuit unit 400 is mounted, and upper ends of the connection pins 311 and 312 protruding upward from the coupling part 370 may be exposed to the outside through the upper cover 380. Thus, upper ends of the connection pins 311 and 312 may pass through the head cover 150 and contact the other ends 131b and 132b of the electrode rods 131 and 132 exposed, respectively.

For example, the first connection pin 311 and the second connection pin 312 may be implemented as a pogo pin having a built in spring.

The upper cover 380 may be installed on the coupling part 370 of the inner case 300 and may be formed of, for example, an insulating material such as silicon.

The upper cover 380 may include a first opening 381 for exposing the first connection pin 311 to the outside and a second opening 382 for exposing the second connection pin 312 to the outside.

The first connection pin 311, the second connection pin 312, and the upper cover 380 may be exposed to the outside through an upper opening of the outer case 210.

An outer case 210 may be installed outside the inner case 300 where the switch 330, the circuit board 350, and the upper cover 380 are assembled, and a battery cap 215 may be coupled to a lower part of the inner and outer cases 300 and 210.

An O-ring (not shown) for sealing and a spring 222 electrically connected to a specific battery terminal may be installed in the battery cap 215.

In addition, a second wall part 230 extending outward may be formed at one end of the upper side of the outer case 210, and at least one second protrusion part 231a or 231b may be formed on an outer circumferential surface of the second wall part 230.

The connection pins 311 and 312 and the upper cover 380 may be positioned inside the second wall part 230. In addition, when the handle part 200 is coupled to the head part 100, the second wall part 230 can be inserted into the accommodating groove 161 of the head body 130, and the second protrusions 231a and 231b may be fastened to at least one insertion groove 135a, 135b formed on the inner surface of the distal end of the head body 130.

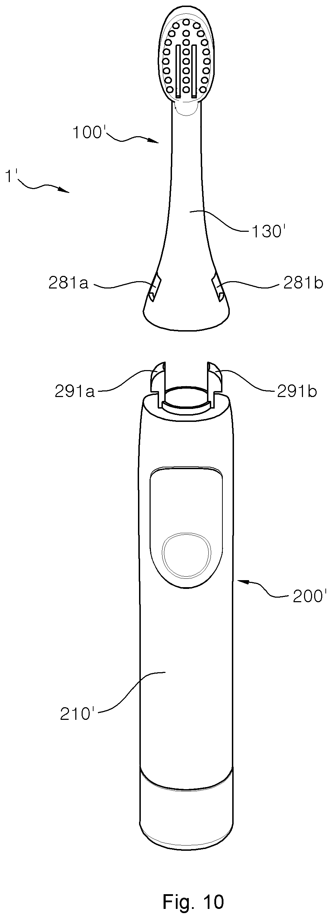

FIG. 10 is a view showing a coupling method of an electric toothbrush according to another embodiment of the present invention.

Referring to FIG. 10, a head part 100' and a handle part 200' of an electric toothbrush 1' according to another embodiment of the present invention may be coupled to fasteners 281a and 281b formed in a head body 130' through extension pieces 291a and 291b formed in an outer case 210 '.

For example, a first fastener 281a and a second fastener 281b may be formed at the distal end of the head body 130', and a first extension piece 291a and a second extension piece 291b may formed on the upper side of the outer case 210'. In this case, the first extension piece 291a and the second extension piece 291b may extend upward from the outer case 210' and have a protruding area at their distal ends.

Accordingly, the user fastens the extension pieces 291a and 291b of the outer case 210' to the corresponding fasteners 281a and 281b of the head body 130', respectively, so that the head part 100' and the handle part 200' may be fixedly coupled.

FIG. 11 is a diagram showing a circuit unit according to an embodiment of the present invention, and FIGS. 12a to 12c are diagrams for explaining driving voltages according to an embodiment of the present invention.

Referring to FIG. 11, the circuit unit 400 according to an embodiment of the present invention may include a DC-DC converter 410, a signal generator 420, a filter 430, and a calibration unit 440.

The DC-DC converter 410 may receive voltage from the battery 310 and convert it to a voltage of a predetermined level.

For example, when the switch 330 is turned on, the DC-DC converter 410 may operate by receiving the battery voltage, and when the switch 330 is turned off, the operation of the DC-DC converter 410 may be stopped.

The signal generator 420 operates based on the voltage supplied from the DC-DC converter 410, and may generate a driving voltage using the output voltage of the DC-DC converter 410.

In this case, the driving voltage may be set to a frequency of 1 KHz to 1,000 MHz. This is because when the driving voltage is set to a low frequency of less than 1 KHz, the removal effect of dental plaque is reduced, and even when the driving voltage is set to a very high frequency exceeding 1,000 MHz, the removal effect of the dental plaque is reduced. Meanwhile, the frequency of the driving voltage may be set to a frequency of 5 MHz to 15 MHz suitable for removing the dental plaque.

The signal generator 420 may be implemented using well-known components such as an oscillator and a function generator.

The filter 430 may perform a filtering operation on the driving voltage generated by the signal generator 420. For example, the filter 430 may include a low pass filter to convert a driving voltage in the form of a saw tooth wave into a sine wave form. However, the type of filter 430 is not limited thereto, and various filters may be employed depending on the design structure.

The calibration unit 440 may perform voltage calibration on the driving voltage supplied from the filter 430 and output the same.

In this case, the calibration unit 440 may receive an alternating current (AC) type of driving voltage and apply a direct current (DC) voltage to the corresponding driving voltage to adjust an offset of the driving voltage.

Accordingly, the driving voltage may be generated by overlapping an alternating current (AC) voltage and a direct current (DC) voltage.

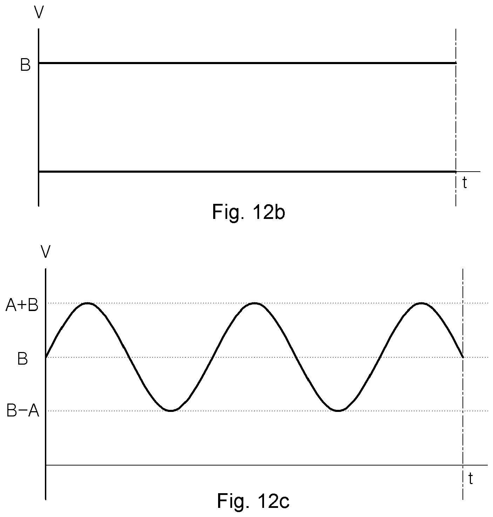

Referring to FIG. 12a, the calibration unit 440 may receive an AC voltage having amplitude of A volt (V) from the filter 430, and by superimposing a DC voltage of B volts (V) as shown in FIG. 12b on the corresponding AC voltage, a final driving voltage as shown in FIG. 12c may be generated.

In this case, the ratio of the amplitude A of the AC voltage and the voltage value B of the DC voltage may be set to 1:0.5 to 10.

For example, when the amplitude A of the AC voltage is set to 0.2V to 0.3V, the voltage value B of the DC voltage may be set to 0. 1V to 3.0V.

As described above, the driving voltage is set in the form of overlapping the AC voltage and the DC voltage, thereby reducing the risk of electric shock and the pain that may cause to the body compared to the case where only the DC voltage is applied. However, it is not limited thereto, and the driving voltage may be composed of only a direct current voltage or an alternating current voltage as needed.

Although not separately shown, a separate controller may be additionally installed in the circuit unit 400.

Meanwhile, the driving voltage supplied from the circuit unit 400 to the head part 100 may be set to a voltage of 0.1 mV to 3 V This is because it is difficult to expect a plaque removal effect when the driving voltage is less than 0.1 mV, and when the driving voltage exceeds 3V, toxic substances may be generated due to electrolysis of bodily fluids.

In addition, the driving voltage may have a form of a pulse wave, a square wave, a triangle wave, or the like, in addition to a sine wave.

Those skilled in the art to which the present disclosure pertains will understand that the present disclosure can be embodied in other specific forms without changing its subject matter or essential features. Therefore, it should be understood that the embodiments described above are illustrative only and not restrictive. The scope of the present disclosure is defined by the claims below rather than the detailed description above, and all changes or modifications derived from the claims and their equivalents should be construed as being included in the scope of the present disclosure.

## Claims

1. An electric toothbrush comprising:
a head part; and
a handle part having a shape that is capable of being coupled to the head part and supplying a driving voltage to the head part according to a user's control;
wherein the head part includes:
a toothbrush head having a plurality of bristle holes in which bristles are disposed and a first through hole and a second through hole spaced apart from each other;
a head body extending from the toothbrush head and having an inner passage connected to the first through hole and the second through hole;
a head cover closing the inner passage of the head body; and
a first electrode rod and a second electrode that are inserted through the inner passage of the head body, have one ends positioned within the first through hole and the second through hole, respectively, and have the other ends exposed to the outside through the head cover, respectively.

2. The electric toothbrush according to claim 1, wherein at least one row of bristle holes are disposed between the first through hole and the second through hole.

3. The electric toothbrush according to claim 1, wherein the width of the first electrode rod and the second electrode rod is 0.1mm to 7mm.

4. The electric toothbrush according to claim 1, wherein one end of the first electrode rod and one end of the second electrode rod are exposed to the outside from the front and rear surfaces of the toothbrush head through the first through hole and the second through hole, respectively.

5. The electric toothbrush according to claim 4, wherein one end of the first electrode rod and one end of the second electrode rod do not protrude outward with respect to the front and rear surfaces of the toothbrush head.

6. The electric toothbrush according to claim 1, wherein the handle part includes
a battery;
a switch for controlling power supply by the battery;
a circuit unit generating a driving voltage using the voltage of the battery; and
a first connection pin and a second connection pin contacting the other end of the first electrode rod and the other end of the second electrode rod, respectively when the head part and the handle part are coupled for transferring the driving voltage generated in the circuit unit to the head part.

7. The electric toothbrush according to claim 6, wherein the driving voltage generated by the circuit unit is set to a frequency of 1 KHz to 1,000 MHz.

8. The electric toothbrush according to claim 7, wherein the circuit unit includes:
a DC-DC converter receiving the voltage of the battery;
a signal generator generating a driving voltage using the output voltage of the DC-DC converter;
a filter performing a filtering operation on the driving voltage generated by the signal generator; and
a calibration unit for performing and outputting voltage calibration for the driving voltage supplied from the filter.

9. The electric toothbrush according to claim 6, wherein the handle part further includes:
an inner case including a battery accommodating part in which the battery is located and a coupling part in which the first connection pin and the second connection pin are installed; and
a circuit board on which the circuit unit is mounted and fixedly installed to the inner case.

10. The electric toothbrush according to claim 9, further comprises:
an upper cover made of an insulating material installed on the coupling part of the inner case and having a first opening and a second opening for exposing the first connection pin and the second connection pin to the outside, respectively;
an outer case accommodating the inner case and having a switch pressing area corresponding to the switch; and
a battery cap coupled to one end of the outer case to close the battery accommodating part.

11. The electric toothbrush according to claim 1, wherein an accommodating groove and a first wall part located inside the accommodating groove and connected to the inner passage are formed at the distal end of the head body.

12. The electric toothbrush according to claim 11, wherein the head cover is fused to the first wall part.

13. The electric toothbrush according to claim 11, wherein at least one first protrusion is formed on the head cover, and
at least one insertion hole to which the first protrusion is coupled is formed in the first wall part.

14. The electric toothbrush according to claim 11, wherein a second wall part extending outward and having at least one second protrusion formed on the outer circumferential surface is formed at one end of the outer case, and
at least one insertion groove into which the second protrusion is inserted and coupled is formed on an inner surface of the distal end of the head body.

15. The electric toothbrush according to claim 1, wherein the driving voltage is generated by superposing an alternating voltage having amplitude of A volt (V) and a direct current voltage of B volt (V).

16. The electric toothbrush according to claim 15, wherein the ratio of the A and the B is set to 1: 0.5 to 10.
